# EUROPEAN PATENT SPECIFICATION

(11) **EP 1 599 197 B1**
(45) Date of publication and mention of the grant of the patent: **11.06.2008**
(21) Application number: 04714246.8
(22) Date of filing: 25.02.2004
(51) Int. Cl.: A61K 31/137, A61P 25/08

(54) **THE USE OF ANTIMUSCARINIC DRUGS**
VERWENDUNG VON ANTIMUSCARINISCHEN ARZNEIMITTELN
UTILISATION DE MEDICAMENTS ANTIMUSCARINIQUES

(30) Priority: 24.02.2003 BR 3031233
(43) Date of publication of application: 30.11.2005
(73) Proprietor: FAPESP-Fundacao de Amparo a Pesquisa do Estado de SP, CEP-05468-901 Sao Paulo-SP (BR); UNIFESP-Universidade Federal de SP, CEP-04023-900 Sao Paulo-SP (BR)
(72) Inventor: MELLO, Luiz Eugênio Araújo de, CEP:05502-60-S o Paulo-SP (BR); BENASSI, Simone Kastropil, CEP 04037-005-S o Paulo-SP (BR); GORGATI, Cristiane, CEP:03513-020-S o Paulo-SP (BR); MASSANT, Cristina Gonçalves, CEP 04141-100-S o Paulo-SP (BR)
(74) Representative: Zinkler, Franz
(86) International application number: PCT/BR2004/000020
(87) International publication number: WO 2004/073583

(56) References cited:
- US-A- 4 945 097
- US-A- 4 988 710
- US-A- 5 037 848

## Description

The current invention relates to a preventive treatment based on the chromic administration of antimuscarinic drugs as a means to prevent the onset of epilepsy that might ensue after a major head injury.

Epilepsy is defined as a condition where motor or non-motor seizures recur even in the absence of a toxic-metabolic or febrile background. The above definition clearly distinguishes between a non-epileptic seizure, which is often a single isolated event associated to a metabolic disturbance or intoxication from the true epilepsies. Even though both epileptic and non-epileptic seizures might have a similar clinical manifestation, a convulsive fit, epilepsy implies a permanently altered background condition. In this manner, the nervous system of persons with epilepsy is in some way different in its anatomy, physiology, and pharmacology from that of persons without epilepsy.

Epilepsy has a high prevalence with an annual incidence ranging from 11 to 131/100,000 and a prevalence of 1.5% in the general population (Guerreiro and Guerreiro, 1993). The etiology of this condition, even though unknown in most cases, can sometimes be clearly linked to a previous lesional event. As such, the incidence of epilepsy as a consequence of severe head trauma ranges from 15 to 80% depending on the extent, location and severity of the injured brain area (Gumnit RJ. Epilepsy and brain injury. In: Epilepsy updated: Causes and treatment, P. Robb (Ed.), Symposia Specialists, Chicago, pp. 177-183, 1980). Indeed the risk for developing epilepsy after severe head trauma is 13 times higher than that of the general population.

Some patients with epilepsy are refractory to the available pharmacological treatment and might be indicated for neurosurgery. However, even in the best medical centers surgical intervention not always results in the complete suppression of seizures (Germano IM, Poulin N and Olivier A. Reoperation for recurrent temporal lobe epilepsy. Journal of Neurosurgery, 81: 31-36, 1994). Indeed, it is possible that in those conditions where the epileptic foci has been completely removed and yet there is still the persistence of seizures after surgery, that the surgical act itself might have constituted a brain trauma capable of triggering epilepsy (Sperling MR, Skolnick J and O'Connor MJ. Prognostic value of auras after temporal lobectomy. Epilepsia 38 (Suppl. 8):80, 1997). As already mentioned severe head traumas are clearly associated to the later onset of epilepsy.

The currently available antiepileptic drugs are able to control seizures in 70% of the persons with epilepsy (Yacubian EMT. *Tratamento medicamentoso das epilepsias.* Lemos Editorial, São Paulo, 1999). Despite this relative success in seizure control, the available medication is not really antiepileptic it is rather antiseizure. The available medication for the treatment of epilepsy does not alter or suppress the underlying epileptic condition but merely suppresses the epileptic seizures. Indeed, to date all of the well controlled clinical trials conducted to evaluate a true antiepileptic agent either in patients that already have epilepsy (capable of suppressing epilepsy) either after severe head trauma (capable of preventing epilepsy) have failed so far (Temkin NR, Dikmen SS and Winn HR. Clinical trials for seizure prevention. In: Antiepileptic drug development. J French, I Leppik and MA Dichter (Eds.), Lippincott-Raven Publishers, Philadelphia, pp. 179-'88, 1998). As a consequence, despite the known risk for developing epilepsy which is present after several conditions associated to lesions of the nervous system, there is no prophylactic strategy currently available (Mello LEAM, O desenvolvimento medicamentoso de novas estruturas moleculares. In: Tratamento medicamentoso das epilepsias. EMT Yacubian (Ed.), Lemos Editorial, São Paulo, pp.107-115, 1999; Temkin et al., 1998 *op*, Cit.).

US Patent No. 5,037,848 discloses biperiden, among other compounds, for reducing the effects of epilepsy, especially lobe epilepsy. Said Compounds were shown to be highly effective in providing protection against the seizures and neurological damage caused by cholinergic neurotoxins, even when administered only after the onset of convulsions.

The current invention is aimed at the prophylaxis of the epilepsies that might ensue after severe head trauma. It is based on results with a model of experimental epilepsy in rats. In a first series of experiments in rats it was observed that the selective destruction of a specific neuronal population that synthesizes acetylcholine (basal forebrain cholinergic neurons) suppressed the later onset of epilepsy. Assuming that the results could had been a consequence of the functional blockade of the basal forebrain cholinergic neurons, a reversible pharmacological antagonist of that system was tested. Thus, we used scopolamine, a cholinergic antagonist, to evaluate its potential in preventing the onset of epilepsy in a model of epilepsy in rats. A use patent for the use of scopolamine salts as agents with the capacity of preventing epileptogenesis was deposited in Brazil in 1999 and subsequently expanded via PCT for 30 different countries.

Therefore, the current invention deals with the use of antimuscarinic drugs, antagonists of the acetylcholine receptor, such as biperiden, that possess a number of advantages over scopolamine.

This drug is a partially reversible selective antagonist of the M1 receptor '(a acetylcholine receptor type) suggesting a more potent action; biperiden is already used in medical practice for the treatment of Parkinson's disease, thus generating a greater likelihood of its use in a different medical condition due to its know safety profile,

The current invention was based on an experimental model of epilepsy in which in rats the induction of a brain lesion is accomplished through the administration of pilocarpine, a cholinergic agonist. According to the original description of this model, approximately 20-30 days after pilocarpine injection in either rats or mice, there is the onset of spontaneous recurrent epileptic seizures (Turski WA, Cavalheiro EA, Schwarz M, Czuczwar SJ, Kleinrok Z and Turski L. Limbic seizures produced by pilocarpine in rats: behavioural, electroencephalographic and neuropathological study. Behavioral Brain Research, 9:315-335, 1983; Mello LEAM, Cavalheiro EA, Babb TL, Kupfer WR, Pretorius JK, Tan AM and Finch. DM. Circuit mechanisms of seizures in the pilocarpine model of chronic epilepsy: cell loss and mossy fiber sprouting. Epilepsia 34:985-995, 1993). Due to its special features the pilocarpine model is currently one of the 3 most widely used experimental models in the study of epilepsy.

Untill now there has been no experimental procedure that has been shown to be effective in preventing the further development of epilepsy in the pilocarpine or in similar models of epilepsy. In the current work with antimuscarinic compounds, in special with biperiden, it was demonstrated that its prophylactic use after pilocarpine lesion significantly retarded or even supressed the onset of spontaneous recurrent seizures, i.e., epilepsy. In those animals that eventually went on and developed epileptic seizures, these were at a significantly lesser frequency, as compared to that of animals not treated with biperiden.

Biperiden, together with atropine, is a classic cholinergic antagonist and is known to block muscarinic receptors. These drugs are known by their abilities in blocking the cholinergic transmission which is otherwise mediated through muscarinic receptors. For our experiments we used Wistar, adult, male, rats which were maintained under controled conditions of temperature, light/dark cylces and kept in groups of up to 6 animals in polypropilene boxes with free access to tap water and rat chow pellets. Rats were subject to the intraperitoneal administration of pilocarpine in a dose of 320 mg/Kg for the induction of status epilepticus.

As expected, the systemic administration of pilocarpine lead to a sequence of behavioral alterations such as akinesia, body tremor and/or myoclonic jerks of the head and oro-facial stereotyped movements accompained by profuse salivation. Status epilepticus, a condition of uninterrupt epileptic seizure, developed on average 19 ± 5 min after the injetion of pilocarpine. Three hours after the onset of status epilepticus the animals in the experimental group (n=10) were intraperitoneally injected with the first initial dose of biperiden (8 mg/kg). This dose represents the concentration needed to provoke memory impairments in rodents (Roldan G, Bolanos-Badillo E, Gonzales-Sanches H, Quirarte GL and Prado-Alcala RA JW. Selective M1 muscarinic receptor antagonists disrupt memory consolidation of inhibitory avoidance in rats. Neurosci. Lett., 230:93-6, 1997).

Biperiden was given every 8 hours, to allow the maintenance of a steady therapeutic level once its half-life is 24 ± 1.2 h during a total tretament period of 10 days. For the animals in the control group (n=6), two hours after the onset of status epileticus, administration of sterile saline solution (0.9% NaCl), rather than biperiden, was started with a similar protocol.

In the pilocarpine model of epilepsy, a few weeks after the administration of pilocarpine and the induction of status epilepticus, the animals develop spontaneous epileptic seizures which reccur for as long as the animal are allowed to live. It is assumed that such epileptic condition adequately mimics some types of epileptic seizures in humans and may closely reflect the sequence of events that take place in post-traumatic epilepsies.

Approximately 15 to 20 days after the induction of status epilepticus, the reported duration of the latent period for the onset of spontaneous seizures, monitoring of the behavioral seizures with a videocamera for approximately 12 hours/week was initiated. Based on our initial hypothesis, that the experimental group (treated with biperiden) would either have a substantial reduction or would show no spontaneous epileptic seizures, and therefore to avoid a false negative result, the sampling rate for video monitoring of these animals was performed for 24 hours/week. Animals were video monitored for approximately 120 days after the induction of status epilepticus. The recorded video tapes were routinelly assessed in a 29 inches wide television screen and the seizures observed for each animal were recorded.

The latency for the initiation of the video recording sessions was 3.2 ± 1.1 weeks for the control group, and thus within the period that has been reported for the spontaneous seizures to initiate, and 6.0 ± 1.1 weeks for the experimental group, thus two times greater than that of the untreated group (Table 1). The encountered values for the control group are strikingly similar to those already reported in the various papers that characterized the pilocarpine model. This similarity therefore help us on validating our procedure for monitoring the spontaneous seizures.

*Table 1.* Length of the latent period for the onset of the first recorded spontaneous epileptic seizure for the experimental and the control group

| | **Latency (weeks)** |
|---|---|
| CONTROL | 3.2 ± 1.1 |
| EXPERIMENTAL | 6.0 ± 1.1* |

| | |
|---|---|
| *Data expressed as mean ± standard deviation; *p < 0. 03 (Mann-Whitney).* | |

As it can be seen, the data expressed in Table 1 clearly indicate a protective effect of the adopted therapy with regard to the onset of spontaneous epiletic seizures in this model. Indeed, the mean latency of the experimental group was twice that of the control group. This effect alone, if replicated in human clinical studies, would already represent a major advance in the control of post-traumatic epilepsies.

The comparison between the control and the experimental groups with regard to seizure frequency did also yield statistically significant differences (see Table 2).

**Table 2. Frequency of spontaneous epileptic seizures for the control and experimental groups.**

| | **Spontaneous seizures/h** |
|---|---|
| CONTROL (n=6) | 0.0401 ± 0.0113 |
| EXPERIMENTAL (n=10) | 0.0202 ± 0.0047* |

| | |
|---|---|
| *Data expressed* as *mean ± standard deviation; *p* < *0.02 (Mann-Whitney).* | |

Table 2 clearly shows that the mean frequency of spontaneous epileptic seizures for the control group was 2 times higher than that for the experimental group (treated with biperiden). For each individual seizure however, we found no differences between the two groups regarding its qualitative or quantitative nature. Therefore, also in this other feature, the treatment with biperiden yielded a protection.

Our results suggest that the chronic administration (e.g., continuous infusion, multiple administrations, transdermic administration) of biperiden for 10 days after a major head injury might have the potential to diminish or block the later development of epilepsy. The possible use of the current invention in humans will naturally have to relie on clinical trials to establish the appropriate dosages and treatment durations for achieving optimal results. The current results support the development of clinical studies with biperiden treatment right after the occurrence of major head trauma in humans. Given the frequency of major head trauma and the strong clinical evidence linking this event with the later development of epilepsy it should not be difficult to recruit the necessary number of patients to perform a preliminary assessment of the suggest therapy. Finally it is important to remember that there is currrently no available therapy that might be used in this condition.

## Claims

1. Use of antimuscarinic drugs for the preparation of a medicament to be chronically applied after head injury for diminishing or blocking the subsequent development of epilepsy.

2. The use of antimuscarinic drugs according to claim 1, wherein such drugs are applied after accidents with major head trauma.

3. The use of antimuscarinic drugs according to claim 1, wherein such drugs are applied after surgeries for the removal of anatomical structures of epileptogenic nature.

4. The use of antimuscarinic drugs according to claim 1, wherein such drugs are applied after episodes of status epilepticus.

5. The use of antimuscarinic drugs according to any preceding claims, wherein such antimuscarinic drug is biperiden.

6. Use of antimuscarinic drugs according to any preceding claims, wherein such antimuscarinic drug is chronically applied for 10 days after injury.

## Patentansprüche

1. Verwendung von Antimuskarinarzneimitteln für die Vorbereitung eines Medikaments, das nach einer Kopfverletzung zum Verringern oder zum Blockieren der nachfolgenden Entwicklung einer Epilepsie chronisch anzuwenden ist.

2. Die Verwendung von Antimuskarinarzneimitteln gemäß Anspruch 1, bei der derartige Arzneimittel nach Unfällen mit größeren Schädeltraumata angewendet werden.

3. Die Verwendung von Antimuskarinarzneimitteln gemäß Anspruch 1, bei der derartige Arzneimittel nach operativen Eingriffen zur Entfernung von anatomischen Strukturen von epileptogener Natur angewendet werden.

4. Die Verwendung von Antimuskarinarzneimitteln gemäß Anspruch 1, bei der derartige Arzneimittel nach Episoden eines Status epilepticus angewendet werden.

5. Die Verwendung von Antimuskarinarzneimitteln gemäß einem der vorhergehenden Ansprüche, bei der ein derartiges Antimuskarinarzneimittel Biperiden ist.

6. Die Verwendung von Antimuskarinarzneimitteln gemäß einem der vorhergehenden Ansprüche, bei der ein derartiges Antimuskarinarzneimittel nach einer Verletzung 10 Tage lang chronisch angewendet wird.

## Revendications

1. Utilisation de médicaments antimuscariniques pour la préparation d'un médicament à appliquer de manière chronique après une lésion crânienne en vue de diminuer ou de bloquer le développement ultérieur d'épilepsie.

2. Utilisation de médicaments antimuscariniques selon la revendication 1, dans laquelle ces médicaments sont appliqués après des accidents avec traumatisme crânien important.

3. Utilisation de médicaments antimuscariniques selon la revendication 1, dans laquelle ces médicaments sont appliqués après des interventions chirurgicales pour éliminer des structures anatomiques de nature épileptogène.

4. Utilisation de médicaments antimuscariniques selon la revendication 1, dans laquelle ces médicaments sont appliqués après des épisodes d'état de mal épileptique.

5. Utilisation de médicaments antimuscariniques selon l'une quelconque des revendications précédentes, dans laquelle ce médicament antimuscarinique est le bipéridène.

6. Utilisation de médicaments antimuscariniques selon l'une quelconque des revendications précédentes, dans laquelle ce médicament antimuscarinique est appliqué de manière chronique pendant 10 jours après la lésion.
